# EUROPEAN PATENT SPECIFICATION

(11) **EP 2 363 499 B1**
(45) Date of publication and mention of the grant of the patent: **02.07.2014**
(21) Application number: 09828668.5
(22) Date of filing: 03.11.2009
(51) Int. Cl.: C12Q 1/68

(54) **COLORECTAL CANCER DIAGNOSTIC METHOD**
DIAGNOSEVERFAHREN FÜR KOLOREKTALKARZINOM
MÉTHODE DE DIAGNOSTIC DE CANCER COLORECTAL

(30) Priority: 03.11.2008 ES 200803127
(43) Date of publication of application: 07.09.2011
(73) Proprietor: Fina Biotech, S.L.U., 28023 Madrid (ES)
(72) Inventor: CAPELLA MUNAR, Gabriel, E-28023 Madrid (ES); AZUARA GARCIA, Daniel, E-28023 Madrid (ES); ESTELLER BADOSA, Manel, E-28023 Madrid (ES)
(74) Representative: Carpintero Lopez, Francisco
(86) International application number: PCT/ES2009/070479
(87) International publication number: WO 2010/061023

(56) References cited:
- WO-A2-2006/119434
- US-A1- 2007 298 431
- XU X L ET AL: "Methylation profile of the promoter CpG islands of 31 genes that may contribute to colerectal carcinogenesis", WORLD JOURNAL OF GASTROENTEROLOGY, WJG PRESS, CN, vol. 10, 1 January 2004 (2004-01-01), pages 3441-3454, XP008084346, ISSN: 1007-9327
- ESTELLER M ET AL: "A GENE HYPERMETHYLATION PROFILE OF HUMAN CANCER", CANCER RESEARCH, AMERICAN ASSOCIATION FOR CANCER RESEARCH, US, vol. 61, 15 April 2001 (2001-04-15), pages 3225-3229, XP002982492, ISSN: 0008-5472
- AHMED FARID E: "Colorectal cancer epigenetics: the role of environmental factors and the search for molecular biomarkers", ENVIRONMENTAL CARCINOGENESIS AND ECOTOXICOLOGY REVIEWS, DEKKER, NEW YORK, NY, US, vol. 25, no. 2, 1 April 2007 (2007-04-01), pages 101-154, XP009143492, ISSN: 1059-0501, DOI: 10.1080/10590500701399184
- RODRIGUEZ-MORANTA F ET AL: "W1990 Stool DNA Melting Curve Analysis of Methylated Promoters Is Sensitive and Specific for the Non-Invasive Early Diagnostic Tool for Colorectal Tumors", GASTROENTEROLOGY, ELSEVIER, PHILADELPHIA, PA, vol. 136, no. 5, 1 May 2009 (2009-05-01), pages A-768, XP026114095, ISSN: 0016-5085, DOI: 10.1016/S0016-5085(09)63548-4 [retrieved on 2009-05-01]
- DANIEL AZUARA ET AL: "Novel Methylation Panel for the Early Detection of Colorectal Tumors in Stool DNA", CLINICAL COLORECTAL CANCER, vol. 9, no. 3, 1 July 2010 (2010-07-01), pages 168-176, XP55036425, ISSN: 1533-0028, DOI: 10.3816/CCC.2010.n.023
- GRADY, W.M. ET AL.: 'Genomic and epigenetic instability in colorectal cancer pathogenesis.' GASTROENTEROLOGY vol. 135, no. 4, October 2008, pages 1079 - 1099, XP025623120
- AHMED, FE.: 'Colorectal cancer epigenetics: the role of environmental factors and the search for molecular biomarkers.' JOURNAL OF ENVIRONMENTAL SCIENCE AND HEALTH PART C ENVIRONMENTAL CARCINOGENESIS & ECOTOXICOLOGY REVIEWS vol. 25, no. 2, 2007, pages 101 - 54, XP009143492
- XU, X.L. ET AL.: 'Methylation profile of the promoter CpG islands of 31 genes that may contribute to colorectal carcinogenesis.' WORLD JOURNAL OF GASTROENTEROLOGY vol. 10, no. 23, 2004, pages 3441 - 3454, XP008084346
- BELSHAW, N.J. ET AL.: 'Use of DNA from human stools to detect aberrant CpG island methylation of genes implicated in colorectal cancer.' CANCER, EPIDEMIOLOGY, BIOMARKERS & PREVENTION vol. 13, 2004, pages 1495 - 501, XP008150426
- LIND, G.E. ET AL.: 'A CpG island hypermethylation profile of primary colorectal carcinomas and colon cancer cell lines.' MOLECULAR CANCER vol. 3, no. 28, 2004, pages 1 - 11, XP008150422
- LEE, S. ET AL.: 'Aberrant CpG island hypermethylation of multiple genes in colorectal neoplasia.' LABORATORY INVESTIGATION vol. 84, no. 7, 2004, pages 884 - 893, XP008150428
- ESTELLER, M. ET AL.: 'Analysis of adenomatous polyposis coli promoter hypermethylation in human cancer.' CANCER RESEARCH vol. 60, no. 16, 2000, pages 4366 - 4371, XP002352434

### SEQUENCE LISTING

<110>FINA BIOTECH S.L.U.
<120>METHOD FOR THE DIAGNOSIS OF COLORECTAL CANCER
   <130> 270/08
<160> 5
<210> 1
   <211> 40
   <212> DNA
   <213> Artificial
<223> Primer 5'-3' RARB2
<400> 1
   tttaaagtgt gggttggggg cccaacaacc ctacaaaac 40
<210> 2
   <211> 41
   <212> DNA
   <213> Artificial
<223> Primer 5'-3' p16
<400> 2
   gttggttatt agagggtggg gctacaaacc ctctaccac c 41
<210> 3
   <211> 38
   <212> DNA
   <213> Artificial
<223> Primer 5'-3' MGMT
<400> 3
   ggatatgttg ggatagttaa ataaataaa atcaaaac 38
<210> 4
   <211> 44
   <212> DNA
   <213> Artificial
<223> Primer 5'-3' p14
<400> 4
   gtttattttt ggtgttaaag ggctaactcc tcaataacat caac 44
<210> 5
   <211> 38
   <212> DNA
   <213> Artificial
<223> Primer 5'-3' APC
<400> 5
   ggttagggtt aggtaggttg ctacaccaat acaaccac 38

## Description

### FIELD OF THE INVENTION

The field of application of the present invention is the medical sector, in the field of Molecular Biology. More specifically, the invention addresses a method for the early diagnosis of colorectal cancer and the kit for performing the method.

### BACKGROUND OF THE INVENTION

Colorectal cancer (CRC) is the third most common form of cancer and the second leading cause of cancer-related deaths in the western world. Its natural evolution (adenoma-carcinoma sequence is believed to occur in most patients) and accessibility by non-surgical methods makes it suitable for early detection and prevention.

Early detection greatly improves the chances of curing CRC. Colonoscopy is an extremely specific and sensitive test, which could be considered the reference method for an early diagnosis of CRC [44,45], and is typically recommended to be performed at 10-year intervals for detection, usually beginning at the age of 50 [1]. However, it is an invasive test which requires good bowel preparation and the patient's cooperation. It is also associated to a minor risk of serious complications, reducing its generalised use.

Fecal occult blood testing (FOBT) is generally considered to be the best non-invasive test for CRC, since it reduces cancer-related mortality when used programmatically [2]. However, its sensitivity is fairly low (15% for adenomas and 35% for carcinomas), implying that a substantial proportion of colorectal neoplasms are missed [1]. Additionally, it has a low specificity giving rise to a lot of false positives. Also, some tests require patients to change their diet, which considerably reduces its usability [46-47].

In this context, DNA testing in non-invasive samples appears as a feasible approach that can complement and probably outperform other screening test for CRC [3-5] [23-26], although to date, its routine clinical application is still a matter of debate [6-7, 27].

DNA appears to be stable in stool, and although PCR inhibitors are present, purification steps have made it possible to carry out PCR-based analyses systematically in a high proportion of samples [28-30]. This proportion is even higher when lesions, especially colorectal carcinomas, are diagnosed. Stool samples in subjects with CRC can contain more exfoliated cells and/or amplifiable DNA [31].

Most of the studies published to date using stool DNA have detected mutations in single genes or markers (e.g., K-*ras*, p53, *APC* or BAT26) [16,26,32-33] with acceptable although variable sensitivity and specificity. Others have postulated the use of a multiple target mutation assay panel, which in a large screening setting, showed a sensitivity of 51.6% for carcinomas and 32.5% for adenomas with high grade dysplasia [29]. These promising results are associated with a moderate reduction in the level of specificity (95%), due to the detection of mutations in endoscopically normal colons.

Methylation of the cytosine residues of CpG-rich sequences (CpG islands), located within the gene promoter regions, is usually associated with gene silencing and is considered as a mechanism for cancer gene inactivation.

The genomes of pre-neoplastic, cancerous and aged cells share three important changes in the methylation levels, as early events in the development of certain tumours. Firstly, the hypomethylation of heterochromatin which leads to genomic instability and increases the events of mitotic recombination; secondly, hypermethylation of individual genes, and finally, hypermethylation of the CpG islands of constitutive genes and tumour suppressant genes. The two levels of methylation can occur individually or simultaneously. In general, hypermethylation is involved in gene silencing and hypomethylation in the over-expression of certain proteins linked to the processes of invasion and metastasis.

Promoter hypermethylation of many genes, such as *p16^{NK4a}, p14^{ARF}, APC, MGMT, LKB1, hMLH1, RASSF1A, CRBP1, RARB2* [8-10], are a common and early events in colorectal tumorigenesis.

In the last few years, changes in the methylation of cytosine residues of CpG-rich sequences (CpG islands) have been increasingly recognised as very common molecular aberrations presumed to be of clinical use [34]. In colorectal cancer, methylated markers are interesting for screening because they occur with high frequency in early stage neoplasia [35]. Detection of CpG islands methylation in human DNA isolated from stool [11-12,36] or serum [37-39] has been proposed as a novel strategy for the early diagnosis of colorectal neoplasia. Thus, the methylation status of the gene promoter regions such as p16, MGMT, MLH1, SFRP2, HIC1 and vimentine in stool DNA has been evaluated, either individually or using a combined panel [11-15,25,40, 48-53]. To date, the methylation of SFRP2 is the most sensitive individual marker (sensitivity of 77-90%). However, both individual and combined methylated markers have been hampered by a relatively high proportion of false positives.

Assesment of promoter hypermethylation in stool DNA could provide a valuable approach to the non-invasively screening for early colorectal lesions [11-15]. Notwithstanding, the lack of specificity demonstrated until now made its potential clinical use difficult. The DNA resulting from the exfoliation of the colonic epithelium in the faecal matter is difficult to amplify given the enormous number of PCR inhibitors found in stools.

Based on the current needs and difficulties in establishing a non invasive early diagnosis of colorectal cancer with acceptable sensitivity and specificity, the authors of the invention have developed a panel of biomarkers of methylation in stool suitable for routine use in clinical practice.

Thus, following considerable research, the authors have found a panel of 5 methylated markers *(RARB2, MGMT, p16^{INK4a}, APC* and *p14^{ARF})* which jointly represent most tumours.

After finding these five markers, the authors of the invention, have developed a new method for the early diagnosis of colorectal cancer based on the analysis of the melting curves through real-time PCR detection (*RT PCR Melting curve,* MC) of the state of methylation of the promoters of the 5 genes: *RARB2, p16^{INK4a}, MGMT, p14^{ARF}* and *APC* in stool DNA, demonstrating high sensitivity in the early detection of CRC tumours and excellent specificity (no false positives in normal colonoscopies).

This new method allows the non invasive detection of pre-malignant and malignant neoplastic alterations of the colon with high sensitivity and excellent specificity, allowing its routine use in clinical practice.

### BRIEF DESCRIPTION OF THE DRAWINGS

- Figure 1: shows the different melting temperatures according to the markers and according to the state of methylation of the sample (M: methylated; U: unmethylated).
- Figure 2: shows the prevalence of methylation in the different markers analysed in the study in the stool DNA (a) and in the tissue (b) in the validation set.

### OBJECT OF THE INVENTION

The object of the present invention refers to an in vitro method for the diagnosis of colorectal cancer based on the determination from a biological sample obtained from a subject, of the promoter methylation status of at least one of the genes APC, RARB2 and p14 and, optionally, MGMT and p16.

Likewise, the object of the present invention is a kit for carrying out the method of the invention.

### DESCRIPTION OF THE INVENTION

A main aspect of the invention contemplates an in vitro method for the diagnosis of colorectal cancer comprising a) obtaining a stool sample from a subject; and b) simultaneously determining by melting curve analysis from the stool sample the methylation status of the promoter of the genes APC, RARB2, p16, MGMT and p14, wherein the methylation of the promoter region of said genes is indicative of said disease.

In the present invention, the term "subject" refers to a human being.

The **APC** gene (adenomatous polyposis coli) (also known as GS; DP2; DP3; BTPS2; DP2.5), located in 5q21-q22, access number NCBI M74088.1, codifies a tumor suppressant protein that acts as an antagonist to the Wnt signalling route. It is also involved in other processes such as cellular migration and adhesion, transcriptional activation and apoptosis. Its deactivation is considered an alteration present in the vast majority of colorectal tumours.

The **RARB** gene (Retinoic acid receptor Beta) (also known as HAP, NR1B2, RRB2), located in 3p24, access number NCBI Y00291.1 codifies a protein belonging to the superfamily of thyroid/steroid hormone receptors of nuclear transcriptional regulators. The gene expresses itself using two different promoters, which have different CpG islands. RARB1 expresses itself mainly during embryogenesis, whereas RARB2 expresses itself in adult tissue and is hypermethylated in carcinogenic cells.

The **p16** gene (cyclin-dependent kinase inhibitor 2A) (CDKN2A), access number L27211.1, is a tumour suppressant gene. Therefore, mutations in this gene increase the risk of developing a wide variety of types of cancer, mostly melanoma. The p16 gene has an alternative reading frame (ARF) which codifies the protein **p14arf**, involved in stabilising p53, and positively regulated by E2F. Both genes are involved in regulating the cell cycle.

The gene **MGMT** (O-6-methylguanine-DNA methyltransferase) is located in 10q26, access number NCBI M29971.1. This gene codifies a protein whose function is to repair DNA lesions, basically in the area of guanine.

In a preferred embodiment, the diagnosis method is based on simultaneous determination of the promoter methylation status of APC, RARB2, p16, MGMT and p14 genes.

In order to determine promoter methylation status of the marker genes, the DNA is extracted from the isolated sample.

In a particular embodiment, following the stool extraction, observing a specific protocol (17), the methylation status of the specified markers is analysed.

Determining the methylation status of the promoters of the 5 genes can be carried out using various techniques.

Methylation-specific PCR (MSP) is a widely used PCR-based technique which selects as a target the methylation status of a limited number of CpG residues with a high sensitivity. However, in line with the previously noted results [41-43], false positives can be observed in both endoscopically normal colons and in patients with Inflammatory bowel disease (IBD). The incomplete conversion following treatment with bisulphite of the CpG in unmethylated samples, in addition to the warping of the amplified alleles and the high analytical sensitivity of the technique, are the reasons behind the appearance of false positives in the results.

On the basis of these limitations, in the present invention methylation is determined by analysing the melting curve (MC). Analysis of the MC of the promoter regions detect simultaneously all CpG residues within an amplified region.

Due to the cytosine-guanine (CG) content following treatment of the sample with sodium bisulphite, those that are methylated will present a higher melting temperature (CG bases join with 3 hydrogen bridge linkages while the adenine-thymine bases (AT) join with 2). This makes it possible to establish for each marker indicative temperatures of whether the sample is methylated or not.

Using the MC technology, the assessment of the panel of methylation markers in the stool DNA identifies pre-malignant and malignant growths with high sensitivity and excellent specificity.

Another main aspect of the invention contemplates a colorectal cancer diagnosis kit for performing the diagnosis method that is the object of this invention. The kit comprises a set of primers (Table 1) suitable for determining in a biological sample the methylation status of the promoter of at least one of the genes APC, RARB2 and p14. In addition, the kit can include primers for determining the state of methylation of the promoter of at least one of the genes p16 and MGMT.

Preferably, the Kit comprises of a set of primers suitable for determining simultaneously the promoter methylation status of the APC, RARB2, p14, MGMT and p16 genes.

### EXAMPLES

### MATERIALS AND METHODS

### Patients and collecting of fecal and tissue samples

### Tumor tissue analyses

A set of biopsies of 250 primary colorectal tumours and paired normal samples were used to evaluate the prevalence of the promoter methylation in 7 different genes (see below).

*Initial set.* Fecal samples were consecutively obtained following a laxative treatment administered to 12 patients recently diagnosed with primary colorectal carcinomas and 20 patients with adenomas prior to a colonoscopy, patients admitted to the Hospital de la Santa Creu i Sant Pau for a colonoscopy under the clinical suspicion of cancer. Paired biopsies of the lesions identified during the colonoscopy and stored as fresh frozen samples.

*Validation set.* Stool samples were collected from 28 patients with sporadic CRC, 23 patients with adenomas, 17 with inflammatory bowel disease (IBD) with colonic location (ulcerative colitis and Crohn colitis) and 20 macroscopically normal subjects undergoing surgery or endoscopy at the University Hospital of Bellvitge. Fecal samples from CRC patients were collected after laxative treatment prior to colonic surgery and during the colonoscopy (bowel lavage) from the remaining patients. Stools were stored at -80°C until processed. All patients gave informed written consent to participate and for their biological samples to be analysed.

### DNA isolation and bisulphite modification

All tissue and stool samples were coded before storage, so that laboratory personnel were not aware of the patients' clinical diagnosis. DNA was extracted from the tissue or cell matter obtained after centrifugation of the bowel lavage or solid stools, as previously described [16-17]. One µg of DNA was chemically modified to convert all non-methylated cytosine to uracil using the EZ DNA methylation-gold kit (Zymo Research, Orange, CA) and eluted in 30 pL of elution buffer. DNA from normal lymphocytes treated in vitro with *Sssl* methyltransferase (New England Biolabs Inc., Beverly, MA) was used as a positive control of the methylated alleles, whereas DNA from normal lymphocytes was used as control for unmethylated alleles.

### DNA methylation analysis

### Methylation-specific PCR (MSP)

Promoter methylation was studied in *RARB2, p16^{INK4a}, MGMT, p14^{ARF} APC, hMLH1* and *LKB1* by using MSP, a method that distinguishes methylated from unmethylated alleles of a given gene [18]. Following the DNA's treatment with bisulphite, which converts the unmethylated cytosines, but not the methylated ones, to uracil, DNA was amplified by PCR using specific primers to methylated and unmethylated sequences. The primers and the PCR conditions for the MSP analysis have been previously described for *RARB2, p16^{INK4a},MGMT, p14^{ARF} APC, hMLH1* and *LKB* [8-9, 19-21].

### Real time PCR temperature dissociation (melting curves)

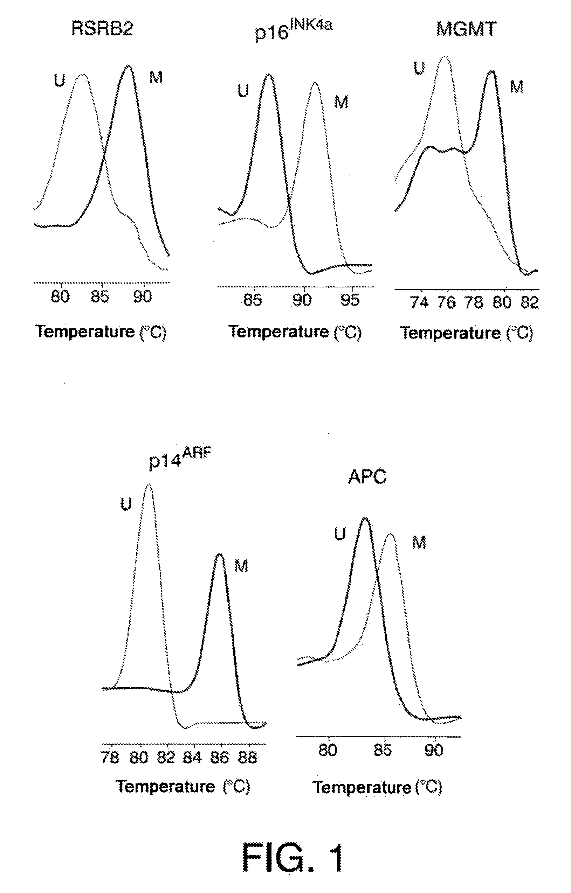

The presence of methylated DNA was detected on the basis of the melting temperature by RT PCR. We analysed the methylation status of 5 genes (*RARB2, p^{16INK4}, MGMT, p14^{ARF}* and *APC*) (figure 1). A PCR was made in all cases in order to obtain a fragment of each one of the CpG islands for analysis (see the conditions in table 1) using a Light Cycler®2.0 (Roche Applied Science) and the Fast Start DNA Master Sybr Green I mix (Roche Applied Science) with real time detection, as described [22].

**Table 1. Sequence of the primers, size of the products, ringing temperature and internal CpGs for the MC tests.**

| Gene | Sequence 5'-3' of the primer | Product size (pb) | Annealing temperature (ºC) | Internal CpGs |
|---|---|---|---|---|
| RARB2 | SEQ ID NO 1 | 273 | 60 | 18 |
| p16^{INK4a} | SEQ ID NO 2 | 254 | 62 | 28 |
| MGMT | SEQ ID NO 3 | 216 | 60 | 22 |
| p14^{ARF} | SEQ ID NO 4 | 224 | 64 | 26 |
| APC | SEO ID NO 5 | 192 | 62 | 16 |

After bisulphite treatment, a methylated sequence conserves the CG pairs whereas an unmethylated sequence presents changes to TG pairs. This change in the CG content gives as a result, a variation in the melting temperature of a particular DNA fragment. Based on the melting temperatures in respect of a series of controls, whose state of methylation was known beforehand, it was possible to assign a state of methylation to the samples undergoing testing. The test of analysing the melting-methylation curve only required one set of primers, which did not include methylation susceptible CpG on its sequence, which increased the reliability of the reaction. This set of primers (table 1) amplified both the methylated and the unmethylated sequence after the treatment with bisulphite. The difference became evident from the melting curves, since the methylated product presented a higher melting temperature than that the unmethylated product.

### RESULTS

### Methylation in CRC tumours by MSP

The prevalence of promoter hypermethylation in tumoral biopsies was as follows: RARB2 (54%, 135 of 250), *p16^{INK4a}* (35%; 88 of 250), *MGMT(35%,* 88 of 250), p*14^{ARF}* (30%, 75 of 50), *APC* (20%, 50 of 250), *hMLH1* (15%, 38 of 250) and *LKB1* (5%, 13 of 250). Informative results were obtained for all tumours when combining all markers analyzed. In 100 cases, paired normal mucosa was analysed, observing a low percentage of methylation: *RARB2* (4%), *MGMT* (4%), *p16^{INK4a}* (2%), *hMLH1* (2%), p*14^{ARF}* (1%), *APC* (0%) and *LKB1* (0%). Finally, no methylation was detected in DNA extracted from the peripheral blood lymphocytes (PBLs) of healthy donors.

### Training set

### Methylation in tissue and fecal DNA by MSP

Taking into account the five most prevalent markers were selected for analysing the stool DNA (RARB, p16, MGMT, p14 and APC). We found that 88% (28 of 32) of the primary tumours (10 of 12 carcinomas and 18 of 20 adenomas) were positive for at least one methylation marker (table 2). Most carcinomas (9 of 10) and a smaller proportion of adenomas (7 of 18) harbored three or more methylated markers. Promoter hypermethylation was found in 25 of 32 (78%) cases for *RARB2,* 18 of 32 (56%) for *MGMT,* 18 of 32 (56%) cases for *p16^{INKa},* 13 of 32 (40%) for *APC* and 10 of 32 (31%) for *p14^{ARF}, p14^{ARF}* and *APC* promoter hypermethylation only increased in carcinomas, when compared to adenomas.

Aberrant methylation of faecal DNA was detected in 1 or more markers in 9 of 12 (75%) carcinomas and in 12 of 20 adenomas (60%). This proportion varied among markers: 92% (12 of 13) for *APC,* 50% (9 of 18) for *p16^{INK4a},* 24% (6 of 25) for *RARB2,* 10% (1 of 10) for *p14^{ARF}* and 11% (2 of 18) for *MGMT.* No aberrant methylation of promters was detected in DNA collected of the 4 tumours that do not present aberrant gene methylation. Sensitivity of the methylation markers for the detection of tumoural cells was 75% for carcinomas and 60% for adenomas (table 2).

**TABLE 2. Prevalence of methylated markers in faecal and biopsy DNA in pairs in the initial group of samples (MSP results).**

| | Faecal DNA | | | Tissue | | |
|---|---|---|---|---|---|---|
| | n | ≥1 methylated gene | ≥2 methylated genes | n | ≥1 methylated gene | ≥2 methylated genes |
| Adenomas | 20 | 12(60%) | 5(25%) | 20 | 18(90%) | 14(70%) |
| Carcinomas | 12 | 9 (75%) | 2(17%) | 12 | 10(83%) | 10(83%) |

### Validation set

### Methylation in tissue and fecal DNA by melting curves

In the first instance, the biopsy and faecal DNA obtained from healthy volunteers and IBD patients was analysed through MSP. An abnormally high prevalence of aberrant methylation in IBD patients in addition to the identification of a large proportion of positive cases in healthy donors, led us to investigate the analysis of melting curves as an alternative to MSP. By means of this approach, at least one methylation marker was detected in 79% of the carcinoma biopsies (22 of 28) and in 70% of the adenomas (16 of 23) (table 3).

**TABLE 3. Prevalence of methylated markers in faecal and biopsy DNA in pairs in the validation group (MC results).**

| | Faecal DNA | | | Tissue | | |
|---|---|---|---|---|---|---|
| | n | ≥1 methylated gene | ≥2 methylated genes | n | ≥1 methylated gene | ≥2 methylated genes |
| Normal | 20 | 0 | 0 | 20 | 0 | 0 |
| IBD | 17 | 2(12%) | 0 | 17 | 3(18%) | 0 |
| Adenomas | 23 | 9(42%) | 2(9%) | 23 | 16(70%) | 7(30%) |
| Carcinomas | 28 | 18(64%) | 7(25%) | 28 | 22(79%) | 9(32%) |

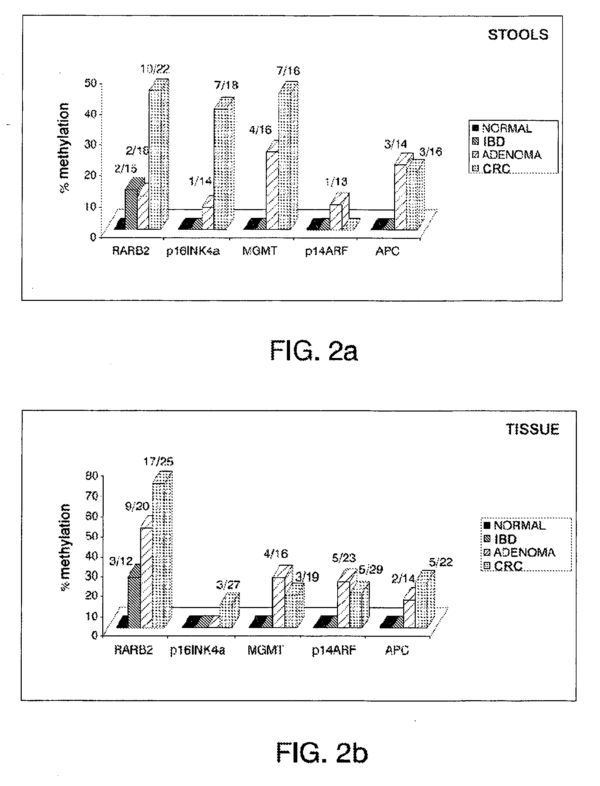

The prevalence for each marker, taking together adenomas and carcinomas, was as follows: 28 of 45 (62%) for *RARB2,* 10 of 51 (20%) for *p14^{ARF},* 7 of 36 (19%) for *APC,* 7 of 35 (20%) for *MGMTand* 3 of 48 (6%) for *p16^{INK4a}.* Three of 12 biopsies (25%) obtained from patients with IBD were positive for *RARE2* (figure 2b)). None of the normal biopsies were positive for any of the analysed markers (table 4).

In respect of faecal DNA, aberrant methylation was detected in 64% of the carcinomas (18 of 28) and in 42% of the adenomas (9 of 23) (table 3), with the distribution of positive markers being as follows: 34% (11 of 32) for *MGMT,* 30% (12 of 40) for *RARB2,* 25% (8 of 32) for *p16^{INK4a}* 20% (6 of 30) for *APC* and 3% (1 of 30) for *p14^{ARF}.* Once more, in 2 of 15 (13%) patients with IBD, *RARB2* promoter hypermethylation was detected (figure 2 a). No methylated marker was detected when the colonoscopy was normal (table 4).

**Table 4. Prevalence of the 5 methylated markers in faecal and biopsy DNA in pairs, in the validation group (MC results).**

| | SAMPLE | p14 | p16 | RARB2 | APC | MGMT |
|---|---|---|---|---|---|---|
| NORMAL | FAECES | 0% | 0% | 0% | 0% | 0% |
| | TISSUE | 0% | 0% | 0% | 0% | 0% |
| INFLAMMATO RY | FAECES | 0% | 0% | 2/15(13%) | 0% | 0% |
| | TISSUE | 0% | 0% | 3/12(25%) | 0% | 0% |
| AD+T | FAECES | 1/30(3%) | 8/32(25 %) | 12/40(30 %) | 6/30(20 %) | 11/32(34 %) |
| | PATHL. TISSUE | 10/51(20 %) | 3/48(6%) | 28/45(62 %) | 7/36(19 %) | 7/35(20) |

The amplification for each marker in healthy individuals was as follows: 15 of 16 (94%) for *MGMT,* 13 of 16 (81%) for *RARB2,* 15 of 20 (75%) for *APC,* 12 of 20 (60%) for *p16^{INK4a}* and 8 of 20 (40%) for *p14*^{*AR*F} *(table 5).*

**Table 5. Amplification of the 5 markers in healthy individuals**

| | SAMPLE | p14 | p16 | RARB2 | APC | MGMT |
|---|---|---|---|---|---|---|
| NORMAL | FAECES | 8/20 (40%) | 12/20(60%) | 13/16(81%) | 15/20(75%) | 15/16(94%) |
| N=20 | TISSUE | 20/20 (100%) | 20/20(100%) | 15/16(94%) | 15/15(100%) | 15/16(94%) |
| INFLAMMATORY | FAECES | 13/17(76%) | 11/15(73%) | 15/17(88%) | 7/11(64%) | 12/15(80%) |
| N=18 | TISSUE | 15/18(100%) | 13/14(93%) | 12/13(92%) | 12/13(92%) | 9/10(90%) |
| | FAECES | 13/23(57%) | 14/23(61%) | 18/18(100%) | 14/19(74%) | 16/17(94%) |
| ADENOMA | TISSUE N | 22/23(96%) | 23/23(100%) | 21/21(100%) | 13/14(92%) | 18/18(100%) |
| N=23 | TISSUE AD | 23/23(100%) | 21/23(91%) | 20/20(100%) | 14/16(88%) | 16/16(100%) |
| | FAECES | 17/30(57%) | 18/30(60%) | 22/23(96%) | 16/21(76%) | 16/21(76%) |
| TUMOUR | TISSUE N | 27/28(96%) | 28/29(97%) | 23/25(92%) | 23/25(92%) | 24/25(96%) |
| N=30 | TISSUE T | 29/30(97%) | 29/30(97%) | 26/27(96%) | 22/25(88%) | 21/21(100%) |

### BIBLIOGRAPHY

1 Winawer S, Fletcher R, Rex D, et al. Colorectal cancer screening and surveillance: clinical guidelines and rationale-Update based on new evidence. Gastroenterology 2003; 124:544-60.
2 Mandel JS, Church TR, Bond JH, et al. The effect of faecaloccult-blood screening on the incidence of colorectal cancer. The New England journal of medicine 2000; 343:1603-7.
3 Dong SM, Traverso G, Johnson C, et al. Detecting colorectal cancer in stool with the use of multiple genetic targets. J Natl Cancer Inst 2001; 93:858-65.
4 Ahlquist DA. Stool-based DNA tests for colorectal cancer: clinical potential and early results. Rev Gastroenterol Disord 2002; 2 Suppl 1:S20-6.
5 Traverso G, Shuber A, Levin B, et al. Detection of APC mutations in faecaIDNA from patients with colorectal tumors. N Engl J Med 2002; 346:311-20.
6 Brenner DE, Rennert G. FaecalDNA biomarkers for the detection of colorectal neoplasia: attractive, but is it feasible? J Natl Cancer Inst 2005; 97:1107-9.
7 Capella G. Is faecalDNA testing superior to faecaloccult-blood testing for colorectal cancer screening? Nat Clin Pract Oncol 2005; 2:234-5.
8 Esteller M, Fraga MF, Guo M, et al. DNA methylation patterns in hereditary human cancers mimic sporadic tumorigenesis. Human molecular genetics 2001; 10:3001-7.
9 Esteller M, Corn PG, Baylin SB, et al. A gene hypermethylation profile of human cancer. Cancer research 2001; 61:3225-9.
10 Esteller M, Guo M, Moreno V, et al. Hypermethylation-associated Inactivation of the Cellular Retinol-Binding-Protein 1 Gene in Human Cancer. Cancer research 2002; 62:5902-5.
11 Belshaw NJ, Elliott GO, Williams EA, et al. Use of DNA from human stools to detect aberrant CpG island methylation of genes implicated in colorectal cancer. Cancer Epidemiol Biomarkers Prev 2004; 13:1495-501.
12 Leung WK, To KF, Man EP, et al. Detection of epigenetic changes in faecaIDNA as a molecular screening test for colorectal cancer: a feasibility study. Clinical chemistry 2004; 50:2179-82.
13 Muller HM, Oberwalder M, Fiegl H, et al. Methylation changes in faecal DNA: a marker for colorectal cancer screening? Lancet 2004; 363:1283-5.
14 Petko Z, Ghiassi M, Shuber A, et al. Aberrantly methylated CDKN2A, MGMT, and MLH1 in colon polyps and in faecalDNA from patients with colorectal polyps. Clin Cancer Res 2005; 11:1203-9.
15 Lenhard K, Bommer GT, Asutay S, et al. Analysis of promoter methylation in stool: a novel method for the detection of colorectal cancer. Clin Gastroenterol Hepatol 2005; 3:142-9.
16 Puig P, Urgell E, Capella G, et al. A highly sensitive method for K-ras mutation detection is useful in diagnosis of gastrointestinal cancer. International journal of cancer 2000; 85:73-7.
17 Puig P, Urgell E, Capella G, et al. Improved detection of K-ras codon 12 mutations in faecalexfoliated cells. Laboratory investigation; a journal of technical methods and pathology 1999; 79:617-8.
18 Herman JG, Graff JR, Myohanen S, et al. Methylation-specific PCR: a novel PCR assay for methylation status of CpG islands. Proceedings of the National Academy of Sciences of the United States of America 1996; 93:9821-6.
19 Herman JG, Umar A, Polyak K, et al. Incidence and functional consequences of hMLH1 promoter hypermethylation in colorectal carcinoma. Proceedings of the National Academy of Sciences of the United States of America 1998; 95:6870-5.
20 Esteller M, Sparks A, Toyota M, et al. Analysis of adenomatous polyposis coli promoter hypermethylation in human cancer. Cancer research 2000; 60:4366-71.
21 Esteller M, Avizienyte E, Corn PG, et al. Epigenetic inactivation of LKB1 in primary tumors associated with the Peutz-Jeghers syndrome. Oncogene 2000; 19:164-8.
22 Frigola J, Song J, Stirzaker C, et al. Epigenetic remodeling in colorectal cancer results in coordinate gene suppression across an entire chromosome band. Nature genetics 2006; 38:540-9.
23 Osborn NK, Ahlquist DA. Stool screening for colorectal cancer: molecular approaches. Gastroenterology 2005; 128:192-206.
24 Song K, Fendrick AM, Ladabaum U. FaecalDNA testing compared with conventional colorectal cancer screening methods: a decision analysis. Gastroenterology 2004; 126:1270-9.
25 Ahlquist DA, Shuber AP. Stool screening for colorectal cancer: evolution from occult blood to molecular markers. Clinica chimica acta; international journal of clinical chemistry 2002; 315:157-68.
26 Traverso G, Shuber A, Olsson L, et al. Detection of proximal colorectal cancers through analysis of faecal DNA. Lancet 2002; 359:403-4.
27 Brenner DE, Rennert G. FaecaIDNA biomarkers for the detection of colorectal neoplasia: attractive, but is it feasible? Journal of the National Cancer Institute 2005; 97:1107-9.
28 Ahlquist DA, Skoletsky JE, Boynton KA, et al. Colorectal cancer screening by detection of altered human DNA in stool: feasibility of a multitarget assay panel. Gastroenterology 2000; 119:1219-27.
29 Imperiale TF, Ransohoff DF, Itzkowitz SH, et al. FaecaIDNA versus faecaloccult blood for colorectal-cancer screening in an average-risk population. The New England journal of medicine 2004; 351:2704-14.
30 Whitney D, Skoletsky J, Moore K, et al. Enhanced retrieval of DNA from human faecalsamples results in improved performance of colorectal cancer screening test. J Mol Diagn 2004; 6:386-95.
31 Loktionov A, O'Neill IK, Silvester KR, et al. Quantitation of DNA from exfoliated colonocytes isolated from human stool surface as a novel noninvasive screening test for colorectal cancer. Clin Cancer Res 1998; 4:337-42.
32 Sidransky D, Tokino T, Hamilton SR, et al. Identification of ras oncogene mutations in the stool of patients with curable colorectal tumors. Science (New York, NY 1992; 256:102-5.
33 Eguchi S, Kohara N, Komuta K, et al. Mutations of the p53 gene in the stool of patients with resectable colorectal cancer. Cancer 1996;77:1707-10.
34 Esteller M. Epigenetics in cancer. The New England journal of medicine 2008; 358:1148-59.
35 Zou H, Harrington JJ, Shire AM, et al. Highly methylated genes in colorectal neoplasia: implications for screening. Cancer Epidemiol Biomarkers Prev 2007; 16:2686-96.
36 Zitt M, Zitt M, Muller HM. DNA methylation in colorectal cancer-impact on screening and therapy monitoring modalities? Disease markers 2007; 23:51-71.
37 Leung WK, To KF, Man EP, et al. Quantitative detection of promoter hypermethylation in multiple genes in the serum of patients with colorectal cancer. Am J Gastroenterol 2005; 100:2274-9.
38 Nakayama G, Hibi K, Kodera Y, et al. P16 methylation in serum as a potential marker for the malignancy of colorectal carcinoma. Anticancer Res 2007; 27:3367-70.
39 Zou HZ, Yu BM, Wang ZW, et al. Detection of aberrant p16 methylation in the serum of colorectal cancer patients. Clin Cancer Res 2002; 8:188-91.
40 Leung WK, To KF, Man EP, et al. Detection of hypermethylated DNA or cyclooxygenase-2 messenger RNA in faecalsamples of patients with colorectal cancer or polyps. The American journal of gastroenterology 2007; 102:1070-6.
41 Cottrell SE, Laird PW. Sensitive detection of DNA methylation. Annals of the New York Academy of Sciences 2003; 983:120-30.
42 Mikeska T, Bock C, El-Maarri O, et al. Optimization of quantitative MGMT promoter methylation analysis using pyrosequencing and combined bisulfite restriction analysis. J Mol Diagn 2007; 9:368-81.
43 Kristensen LS, Mikeska T, Krypuy M, et al. Sensitive Melting Analysis after Real Time- Methylation Specific PCR (SMART-MSP): high-throughput and probe-free quantitative DNA methylation detection. Nucleic acids research 2008; 36:e42.
44 Lieberman DA, Weiss DG, Bond JH, et al. Use of colonoscopy to screen asymptomatic adults for colorectal cancer. Veterans Affairs Cooperative Study Group 380. The New England journal of medicine 2000; 343:162-8.
45 Imperiale TF, Wagner DR, Lin CY, et al. Risk of advanced proximal neoplasms in asymptomatic adults according to the distal colorectal findings. The New England journal of medicine 2000; 343:169-74.
46 Ore L, Hagoel L, Lavi I, et al. Screening with faecal occult blood test (FOBT) for colorectal cancer: assessment of two methods that attempt to improve compliance. Eur J Cancer Prev 2001; 10:251-6.
47 Shields HM, Weiner MS, Henry DR, et al. Factors that influence the decision to do an adequate evaluation of a patient with a positive stool for occult blood. The American journal of gastroenterology 2001; 96:196-203.
48 Markovitz Sanford, D (UNIV CASE WESTERN RESERVE) Methods and compositions for detecting cancers. EP1551437, July 13, 2005. App. 20030757461, June 5, 2003.
49 Hoon Dave S B et al. (WAYNE JOHN CANCER INST). DNA Markers for management of Cancer. WO2004086949, October 14, 2004.
50 Lofton-Day Cathy et al. (EPIGENOMICS AG; LOFTON-DAY CATHY). Methods and nucleic acids for the analysis of colon cell proliferative disorders. WO2005001140, January 6, 2005.
51 Todd Alison Velyian et al. (Johnson & Johnson Res Pty LTD). Method for detection of alkylated cytosine in DNA. EP1644519, April 12, 2006. Appl. 20040737521, July 5, 2004.
52 Lofton-Day Cathy et al. (EPIGENOMICS AG; LOFTON-DAY CATHY; EBERT MATHIAS). Methods and nucleic acids for the detection of metastasis of colon cell proliferative disorders. WO 2006002344, January 5, 2006.
53 Shuber Anthony P. Methods for detecting hypermethylated nucleic acid in heterogeneous biological samples. US6818404, May 8, 2003. Appl. US20020123071, April 11, 2002.

## Claims

1. In vitro method for the diagnosis of colorectal cancer comprising:
A. obtaining a stool sample from a subject; and
B. determining by melting curve analysis from the stool sample from said subject the promoter methylation status of the genes APC, RARB2, p16, MGMT and p14 simultaneously.

2. Method, according to claim 1, **characterised in that** the determination of methylation is carried out on faecal DNA.

## Patentansprüche

1. In vitro Verfahren zur Diagnose eines kolorektalen Karzinoms, umfassend:
A. Nehmen einer Stuhlprobe von einer Testperson; und
B. Simultanes Determinieren des Promotor-Methylierungs-Zustands der Gene APC, RARB2, p16, MGMT und p14 durch Melting-Curve-Analyse der Stuhlprobe von der Testperson.

2. Verfahren nach Anspruch 1, **dadurch gekennzeichnet, dass** die Determinierung der Methylierung auf fäkale DNA angewandt wird.

## Revendications

1. Procédé de diagnostic in vitro du cancer colorectal comprenant les étapes consistant à :
A. obtenir un échantillon de selles d'un sujet, et
B. déterminer par analyse des courbes de fusion de l'échantillon de selles du sujet l'état de méthylation du promoteur des gènes APC, RARB2, p16, MGMT et p14 simultanément.

2. Procédé conforme à la revendication 1,
**caractérisé en ce que**
la détermination de la méthylation est mise en oeuvre sur l'ADN fécal.
